# EUROPEAN PATENT APPLICATION

(11) **EP 3 207 891 A1**
(43) Date of publication of application: **23.08.2017**
(21) Application number: 16811342.1
(22) Date of filing: 09.05.2016
(51) Int. Cl.: A61B 18/12

(54) **POWER SOURCE DEVICE FOR HIGH-FREQUENCY TREATMENT INSTRUMENT, HIGH-FREQUENCY TREATMENT SYSTEM, AND OPERATION METHOD FOR HIGH-FREQUENCY TREATMENT SYSTEM**

(30) Priority: 19.06.2015 JP 2015123625
(71) Applicant: Olympus Corporation, Hachioji-shi, Tokyo 192-8507 (JP)
(72) Inventor: SUGAWARA, Tateyuki, Hachioji-shi, Tokyo 192-8507 (JP)
(74) Representative: Gunzelmann, Rainer
(86) International application number: PCT/JP2016/063772
(87) International publication number: WO 2016/203867

(57) **Abstract**

A power supply device (100) for a high frequency treatment instrument to treat living tissue includes an output unit (140) to supply high frequency power to the treatment instrument's electrode, a distance information acquisition unit (112) to acquire a distance between the living tissue and the electrode, a determination unit (116) to determine whether the distance satisfies a first condition, and an output control unit (118) to control the output unit so that its output is placed in a controlled state if the distance satisfies the first condition and so that the output is set to a first output level higher than an output level in the controlled state if a second condition is satisfied after the output is placed in the controlled state.

## Description

### Technical Field

The present invention relates to a power supply device for a high frequency treatment instrument, a high frequency treatment system, and an operation method for a high frequency treatment system.

### Background Art

Generally, in surgical operations, a high frequency treatment instrument utilizing a high frequency current is used for incision and hemostasis of living tissue. For example, Jpn. Pat. Appln. KOKAI Publication No. 11-290334 discloses a monopolar-type electrical surgical apparatus which comprises an electrode-bearing handpiece and a return electrode, and treats living tissue by causing a high frequency current to flow between the electrode of the handpiece and the return electrode. For such a high frequency treatment instrument, a preset electric power is output from a power supply device to the handpiece upon an operator pressing a push-button switch provided at part of the grip portion of the handpiece.

When such a high frequency treatment instrument is in use, a user turning on the output switch is not limited to only during the state where the electrode of the handpiece is in contact with living tissue as a treatment subject. For example, a user may separate the electrode of the handpiece from the living tissue while the output switch is turned on. It has been known that in such instances an unintentionally large electric discharge can occur when a distance between the electrode and the living tissue reaches a particular distance.

### Summary of Invention

In order to control a high frequency treatment instrument with precision, it is beneficial to have a capability of detecting the state of an electrode being moved away from living tissue.

The present invention purposes to provide a power supply device for a high frequency treatment instrument, a high frequency treatment system, and an operation method for a high frequency treatment system, to control output while automatically detecting that an electrode moves away from living tissue.

According to one embodiment of the present invention, a power supply device is a power supply device for a high frequency treatment instrument which treats living tissue by supplying high frequency power to the living tissue using an electrode, the power supply device comprising: an output unit which supplies the high frequency power to the electrode; a distance information acquisition unit which acquires distance information for a distance between the living tissue and the electrode; a determination unit which determines whether or not the distance information satisfies a first condition when the distance between the living tissue and the electrode is increasing; and an output control unit which controls the output unit so that output by the output unit is placed in a controlled state if the distance information satisfies the first condition and so that the output is set to a first output level higher than an output level in the controlled state if a second condition is satisfied after the output is placed in the controlled state.

According to one embodiment of the present invention, a high frequency treatment system comprises the power supply device and the high frequency treatment instrument.

According to one embodiment of the present invention, an operation method for a high frequency treatment system is a method for operating a high frequency treatment system that treats living tissue by supplying high frequency power to the living tissue using an electrode, the method comprising: supplying, by an output unit, the high frequency power to the electrode; acquiring, by a distance information acquisition unit, distance information for a distance between the living tissue and the electrode; determining, by a determination unit, whether or not the distance information satisfies a first condition when the distance between the living tissue and the electrode is increasing; and controlling, by an output control unit, output of the high frequency power so that the output is placed in a controlled state if the distance information satisfies the first condition, and so that the output is set to a first output level higher than an output level in the controlled state if a second condition is satisfied after the output is placed in the controlled state.

The present invention can provide a power supply device for a high frequency treatment instrument, a high frequency treatment system, and an operation method for a high frequency treatment system, which are capable of carrying out control while detecting that an electrode moves away from living tissue.

### Brief Description of Drawings

FIG. 1 is a block diagram showing an overview of a configuration example of a high frequency treatment system according to one embodiment.
FIG. 2 is an external view of one example of a high frequency treatment system.
FIG. 3 is a figure for explaining an overview of one example of the changes in impedance value with respect to elapsed time when an electrode is moved away from living tissue.
FIG. 4A is a figure for explaining a state of an electrode being moved away from living tissue, in which state the electrode and the living tissue are in contact with each other.
FIG. 4B is a figure for explaining a state of an electrode being moved away from living tissue, in which state the electrode and the living tissue are close to each other and an electric discharge has occurred.
FIG. 4C is a figure for explaining a state of an electrode being moved away from living tissue, in which state the electrode and the living tissue are sufficiently distant from each other.
FIG. 5A is a flowchart showing one example of the operation of a power supply device according to one embodiment.
FIG. 5B is a flowchart showing one example of the operation of a power supply device according to one embodiment.
FIG. 6 is a figure for explaining the updating of a minimum value of impedance.
FIG. 7 shows a table for explaining the updating of a minimum value of impedance.
FIG. 8 is a figure for explaining one example of the changes in measured impedance with respect to time and the accompanying changes of output levels.
FIG. 9 is a figure for explaining another example of the changes of output levels with respect to time.
FIG. 10 is a figure for explaining another example of the changes of output levels with respect to time.
FIG. 11 is a figure for explaining another example of the changes of output levels with respect to time.
FIG. 12 is a figure for explaining another example of the changes of output levels with respect to time.
FIG. 13A is a flowchart showing another example of the operation of a power supply device according to one embodiment.
FIG. 13B is a flowchart showing another example of the operation of a power supply device according to one embodiment.
FIG. 14 is an external view of one example of a high frequency treatment system according to a modification example.
FIG. 15 is a block diagram showing an overview of a configuration example of a high frequency treatment system according to a modification example.

### Description of Embodiments

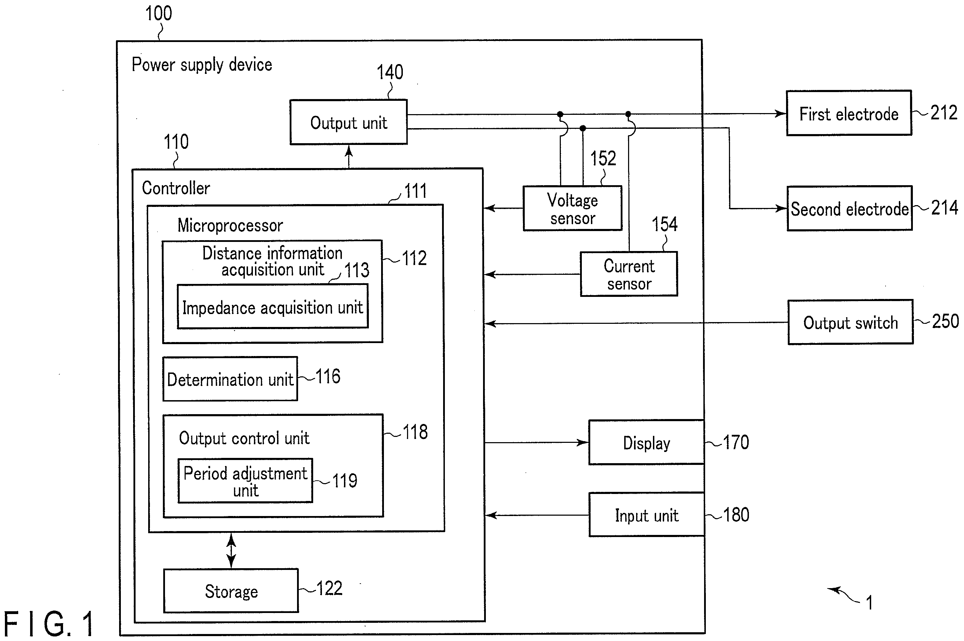

Certain embodiments of the present invention will be described with reference to the drawings. FIG. 1 shows an overview of the configuration example of a high frequency treatment system 1 according to this embodiment. As shown in FIG. 1, the high frequency treatment system 1 is a system for treating living tissue as a treatment subject using high frequency power. The high frequency treatment system 1 comprises a first electrode 212 and a second electrode 214. In the high frequency treatment system 1, living tissue as a treatment subject will be placed between the first electrode 212 and the second electrode 214. By the application of a high frequency voltage between the first electrode 212 and the second electrode 214, a high frequency current flows through and generates heat in the living tissue as a treatment subject so that the treatment of the living tissue such as incision or hemostasis is performed.

The high frequency treatment system 1 comprises a power supply device 100 which supplies electric power between the first electrode 212 and the second electrode 214. The power supply device 100 comprises a controller 110, an output unit 140, a voltage sensor 152, a current sensor 154, a display 170, and an input unit 180.

The controller 110 controls operations of each component of the power supply device 100. The controller 110 includes a microprocessor 111 which is, for example, a central processing unit (CPU) or an application specific integrated circuit (ASIC). The controller 110 may be constituted by one CPU, etc. or a combination of multiple CPUs, ASICs, or the like. The controller 110 operates in accordance with, for example, a program stored in a storage 122 described later.

The output unit 140 outputs electric power supplied to the first electrode 212 and the second electrode 214 under the control of the controller 110. The electric power output from the output unit 140 is supplied to the first electrode 212 and the second electrode 214.

The voltage sensor 152 acquires a voltage value at, for example, the output terminals of the power supply device 100. The voltage sensor 152 transmits the acquired voltage value to the controller 110. The current sensor 154 acquires a current value of an electric current output from, for example, the power supply device 100. The current sensor 154 transmits the acquired current value to the controller 110.

The display 170 includes a display element. The display 170 displays various information concerning the power supply device 100.

The input unit 180 includes a switch, a keyboard, a touch panel, etc. A user gives various inputs to the power supply device 100 using the input unit 180.

The high frequency treatment system 1 further comprises an output switch 250. The output switch 250 is for switching on and off the output of the power supply device 100. The output switch 250 may be provided in a treatment instrument that comprises the first electrode 212, etc., or may be provided as a separate member from the treatment instrument, for example, as a foot switch.

The microprocessor 111 of the controller 110 fulfills the functions of a distance information acquisition unit 112, a determination unit 116, and an output control unit 118. The distance information acquisition unit 112 acquires distance information relating to the distance between living tissue as a treatment subject and an electrode, e.g., the first electrode 212. In this embodiment, the distance information acquisition unit 112 includes an impedance acquisition unit 113. The impedance acquisition unit 113 calculates a value of impedance of, for example, a circuit including the first electrode 212 and the second electrode 214, based on the voltage value acquired by the voltage sensor 152 and the current value acquired by the current sensor 154. This impedance value can be used as an index of the distance between living tissue and an electrode, e.g., the first electrode 212.

The determination unit 116 determines whether or not to temporarily reduce the output, as will be described later, based on the distance between living tissue and an electrode, e.g., the first electrode 212, of a treatment instrument, having been acquired by the distance information acquisition unit 112.

The output control unit 118 controls electric power output from the output unit 140. The output control unit 118 includes a period adjustment unit 119. The period adjustment unit 119 adjusts a period for the temporal reduction of the output as above.

The controller 110 also comprises the storage 122. The storage 122 stores, for example, programs and various parameters for the operations of the microprocessor 111. Also, the storage 122 temporarily stores information necessary for the calculations by the microprocessor 111.

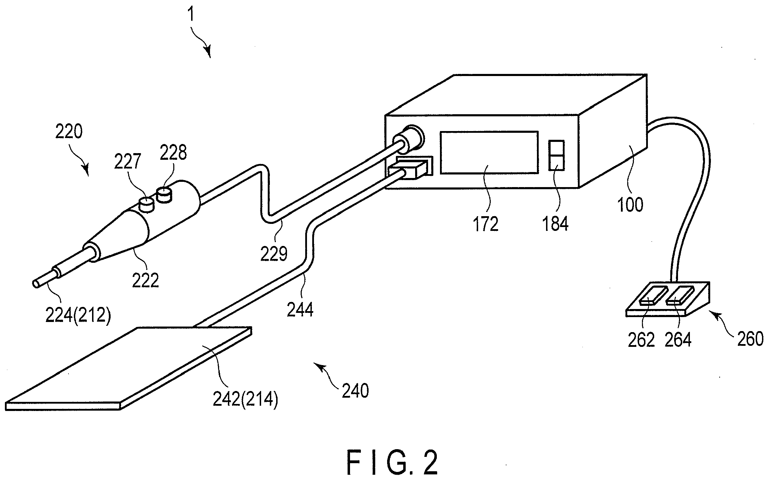

As one example of the high frequency treatment system 1, FIG. 2 shows an external view of the configuration example of a high frequency treatment system including a monopolar-type high frequency treatment instrument. As shown in FIG. 2, the high frequency treatment system 1 comprises the power supply device 100, a high frequency treatment instrument 220, a return electrode unit 240, and a foot switch 260.

The high frequency treatment instrument 220 comprises an operation unit 222, an electrode 224, a first switch 227, a second switch 228, and a first cable 229. The operation unit 222 is a portion for a user to hold and to operate the high frequency treatment instrument 220. The first cable 229 connecting to the operation unit 222 is a cable for the connection between the high frequency treatment instrument 220 and the power supply device 100. The first switch 227 and the second switch 228 function as the output switch 250. The first switch 227 and the second switch 228 are provided at the operation unit 222. The electrode 224 is provided at the distal end of the operation unit 222. This electrode 224 functions as the first electrode 212 described above. During treatment, the electrode 224 is applied to living tissue as a treatment subject.

The return electrode unit 240 comprises a return electrode 242 and a second cable 244. The return electrode 242 functions as the second electrode 214 described above. The second cable 244 is a cable for the connection between the return electrode 242 and the power supply device 100. The return electrode 242 is affixed to a body surface of a person to be treated.

The first switch 227 of the high frequency treatment instrument 220 is a switch for an input to cause the power supply device 100 to output in an incision mode. The incision mode is a mode to burn and cut living tissue as a treatment subject at the portion contacting the electrode 224, with a supply of a relatively large electric power. The second switch 228 is a switch for an input to cause the power supply device 100 to output in a hemostasis mode. The hemostasis mode is a mode to perform hemostatic treatment, with a supply of smaller electric power than in the incision mode, by burning and cutting living tissue as a treatment subject while biologically denaturalizing an end section thereof at the portion contacting the electrode 224.

Also, the foot switch 260 comprises a first switch 262 and a second switch 264. The first switch 262 of the foot switch 260 has the same function as the first switch 227 provided at the high frequency treatment instrument 220. Also, the second switch 264 of the foot switch 260 has the same function as the second switch 228 provided at the high frequency treatment instrument 220. In other words, a user can perform on/off switching of the output of the high frequency treatment instrument 220 using the first switch 227 and the second switch 228 provided at the high frequency treatment instrument, and also using the first switch 262 and the second switch 264 of the foot switch 260.

The power supply device 100 is provided with a display panel 172 and switches 184. The display panel 172 functions as the display 170 described above. That is, it displays various information concerning the state of the power supply device 100. The switches 184 function as the input unit 180 described above. That is, a user uses the switches 184 to input, for example, a setup value for the output such as output electric power, a setup value to define cutting performance called an effect, and so on, to the power supply device 100.

When the high frequency treatment system 1 is in use, a user as an operator brings the electrode 224 into contact with a treatment subject site while, for example, pressing down the first switch 227 or the second switch 228 of the high frequency treatment instrument 220. At this time, an electric current output from the power supply device 100 flows between the electrode 224 and the return electrode 242. As a result, incision or hemostasis of the living tissue is performed at the portion contacting the electrode 224.

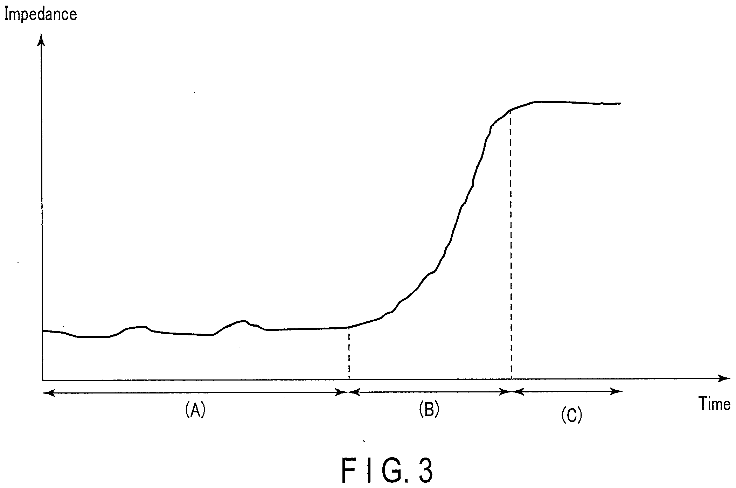

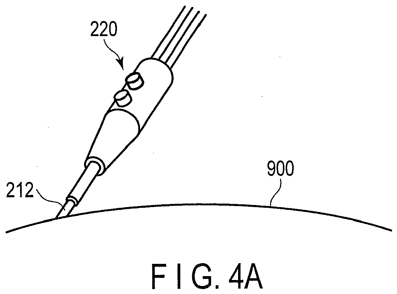

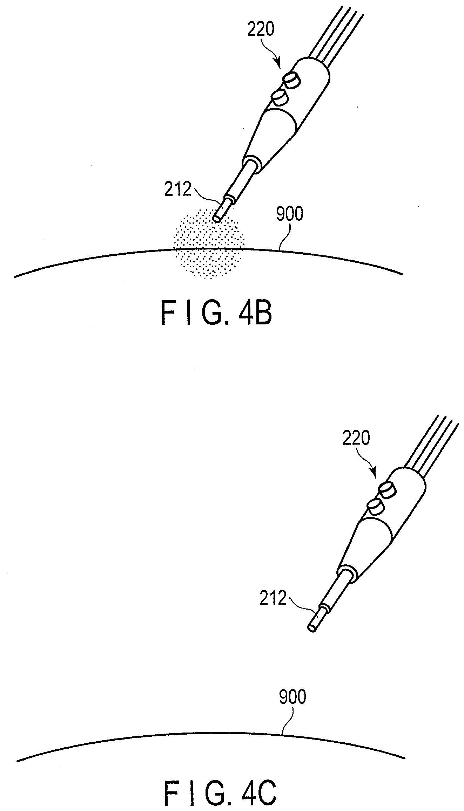

Descriptions will be made with reference to FIG. 3, FIG. 4A, FIG. 4B, and FIG. 4C regarding an overview of the operations of the power supply device 100 according to this embodiment. In FIG. 3, the horizontal axis shows time, and the vertical axis shows impedance of a circuit involving the first electrode 212 and the second electrode 214, calculated by the impedance acquisition unit 113. FIG. 3 illustrates a relationship between time and impedance when the first electrode 212, e.g., an electrode of a monopolar-type high frequency treatment instrument, is gradually moved away from living tissue as a treatment subject from their contact state, while a high frequency voltage is applied between electrodes. During a period indicated as (A) in FIG. 3, the first electrode 212 contacts living tissue 900 as shown in FIG. 4A. In this instance, the impedance value becomes relatively low. During the period indicated as (B) in FIG. 3, the first electrode 212 is moved away from the living tissue 900 and an electric discharge occurs between the first electrode 212 and the living tissue 900. Along with this electric discharge, the acquired impedance rises beyond the impedance measured during the period indicated as (A) in FIG. 3. FIG. 4B schematically shows the state at a certain time point included in the period indicated as (B) in FIG. 3. Between the first electrode 212 and the living tissue 900, for example, the shaded region in the figure, an electric discharge is recognized. During a period indicated as (C) in FIG. 3, there is a sufficient distance between the living tissue 900 and the first electrode 212 as shown in FIG. 4C. In this instance, impedance takes a value under an open condition.

Here, it has been known that during a portion of the period indicated as (B) in FIG. 3, that is, for a certain duration where an electric discharge is present between the first electrode 212 and the living tissue 900 (where the distance between the first electrode 212 and the living tissue 900 has fallen within a particular range to cause a large electric discharge), control can be rendered unstable for the unintentional occurrence of excessive output current flow, or the like. Accordingly, in this embodiment, the output is halted for a portion of the period indicated as (B) in FIG. 3.

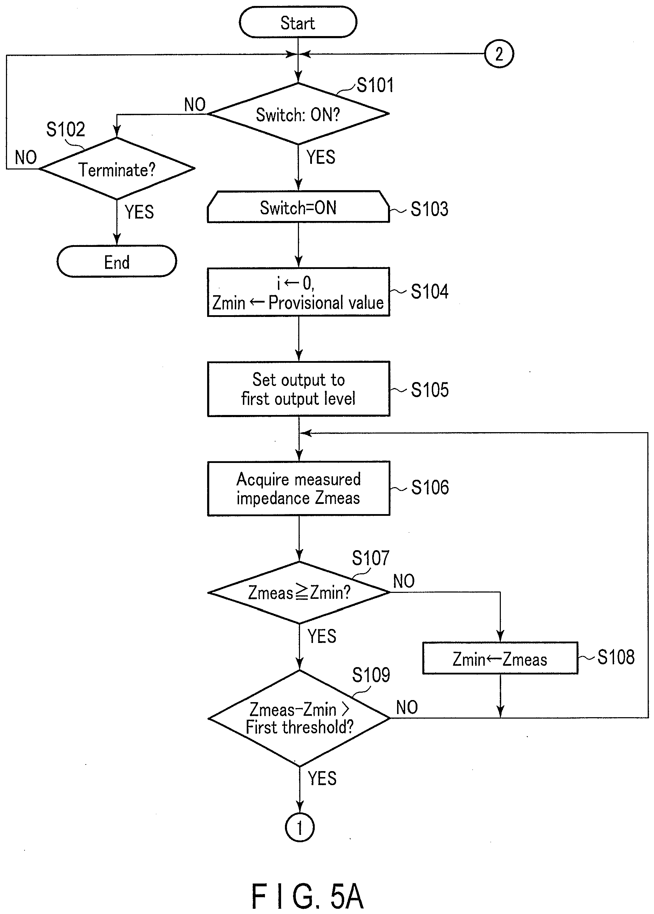

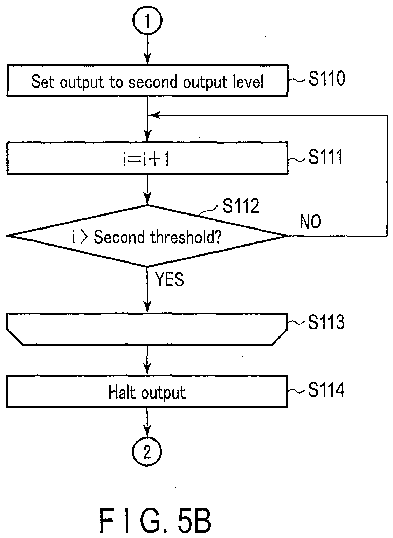

The operations of the power supply device 100 according to this embodiment will be described with reference to the flowcharts shown in FIG. 5A and FIG. 5B. The processing is carried out upon, for example, turning on of a main power of the power supply device 100.

At step S101, the controller 110 determines whether or not the output switch 250 for the on/off instruction of the output is turned on. If it is not on, the processing proceeds to step S102. At step S102, it is determined whether or not the processing is to be terminated, for example, the main power is shut off. If termination is determined, the processing ends. On the other hand, if termination is not determined, the processing returns to step S101. That is, for the off period of the output switch 250, the processing repeats step S101 and step S102 for standby. On the other hand, if it is determined at step S101 that the output switch is on, the processing proceeds to step S103.

The processing from step S103 to step S113 is repetitive processing. The condition for repeating is that the output switch 250 is on. When the output switch 250 is turned off, the processing comes out of this repetitive processing and proceeds to step S114.

At step S104, the controller 110 initializes variables stored in the storage 122. In other words, it sets a later-described first counter i for obtaining a blanking period to zero. It also sets a later-described minimum value Zmin of impedance to a provisional value. Note that the provisional value here is preferably a value which is sufficiently larger than a value expected to be the minimum value Zmin.

At step S105, the controller 110 sets the level of output from the output unit 140 to a first output level. The first output level here is an output level which is, for example, set by a user, and is required for the treatment. Output control may be performed through voltage control, current control, or other methods. Since the output level is set to the first output level, a user can treat the living tissue by allowing the first electrode 212 to contact the living tissue 900.

At step S106, the controller 110 acquires, as measured impedance Zmeas, the impedance of the circuit for the first electrode 212 and the second electrode 214 based on the voltage value acquired by the voltage sensor 152 and the current value acquired by the current sensor 154, etc.

At step S107, the controller 110 determines whether or not the measured impedance Zmeas is equal to or larger than the impedance minimum value Zmin presently stored in the storage 122. If the measured impedance Zmeas is smaller than the minimum value Zmin, the processing proceeds to step S108.

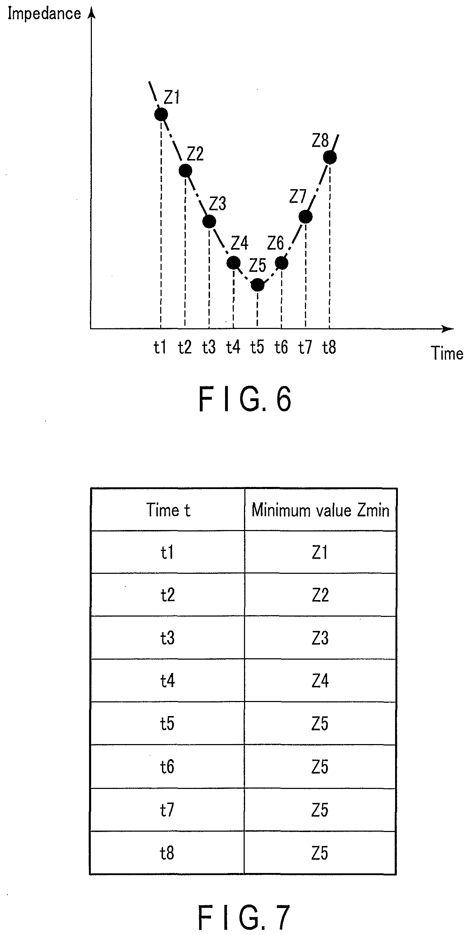

At step S108, the controller 110 substitutes the value of the measured impedance Zmeas for the minimum value Zmin. That is, the minimum value Zmin is updated. The measured impedance Zmeas may not always rise in a uniform manner, but can rise and fall. Accordingly, the impedance minimum value Zmin here is configured to be updated as in the processing at step S108. For example, let us assume that the measured impedance Zmeas gradually falls at Z1, Z2, Z3, Z4, and Z5 along with the passage of time t at t1, t2, t3, t4, and t5, as shown in FIG. 6. In this instance, the impedance minimum value Zmin gradually falls at Z1, Z2, Z3, Z4, and Z5 as shown in FIG. 7. After the processing at step S108, the processing returns to step S106.

At step S107, if the measured impedance Zmeas is determined to be equal to or larger than the minimum value Zmin, the processing proceeds to step S109. For example, it is assumed that the measured impedance Zmeas gradually rises at Z5, Z6, Z7, and Z8 along with the passage of time t at t5, t6, t7, and t8, as shown in FIG. 6. In this instance, the impedance minimum value Zmin remains Z5 without being updated.

At step S109, the controller 110 determines whether or not a difference Zmeas-Zmin obtained by subtracting the impedance minimum value Zmin from the measured impedance Zmeas is larger than a predetermined first threshold. If the difference Zmeas-Zmin is not larger than the first threshold, the processing returns to step S106.

For example, when a user gradually brings the first electrode 212 away from the living tissue 900, the minimum value Zmin remains unchanged while the difference Zmeas-Zmin gradually increases.

If at step S109 the difference Zmeas-Zmin is determined to be larger than the first threshold, the processing proceeds to step S110. The condition that the difference Zmeas-Zmin is larger than the first threshold as above corresponds to a first condition. At step S110, the controller 110 sets the level of output from the output unit 140 to a second output level. Descriptions here are on the assumption that the second output level is zero, but as a matter of course, it may be other than zero. When zero output is adopted, the controller 110 halts the output.

At step S111, the controller 110 increases the first counter i stored in the storage 122.

At step S112, the controller 110 determines whether or not the first counter i is larger than a predetermined second threshold. If the first counter i is not larger than the second threshold, the processing returns to step S111. That is, the processing at step S111 and step S112 is repeated until the first counter i exceeds the second threshold. In other words, the processing waits for a certain period. This period measured by the first counter i, that is, the period for which the output is halted, will be called a blanking period. The blanking period is, for example, 10 milliseconds. The state in the blanking period, where the output level is the second output level as above, corresponds to a controlled state which is a state of output when the distance information satisfies the first condition.

At step S112, if the first counter i is determined to be larger than the second threshold, the processing proceeds to step S113. The condition that the first counter i is larger than the second threshold as above corresponds to a second condition. Here, when the switch is on, the processing from step S103 is repeated.

After the first condition is satisfied and the second output level is set in the period (B) shown in FIG. 3, the processing starting from step S103 is conducted again, and thus, the output of the output unit 140 is again set to the first output level at step S105. The minimum value Zmin of impedance is again set to the provisional value at step S104, so the difference between the measured impedance Zmeas and the minimum value Zmin does not exceed the first threshold. Therefore, the processing repeats the processing from step S106 to step S109. In other words, for the switch-on period, the output at the first output level will be maintained. Since the output at the first output level is maintained, a user can treat the living tissue 900 by allowing the first electrode 212 to contact the living tissue 900 again. The treatment of the living tissue 900 may be performed while repeating the contact and the separation between the first electrode 212 and the living tissue 900.

When the output switch 250 is turned off, the processing proceeds to step S114. At step S114, the controller 110 causes the output unit 140 to halt the output. The processing then returns to step S101.

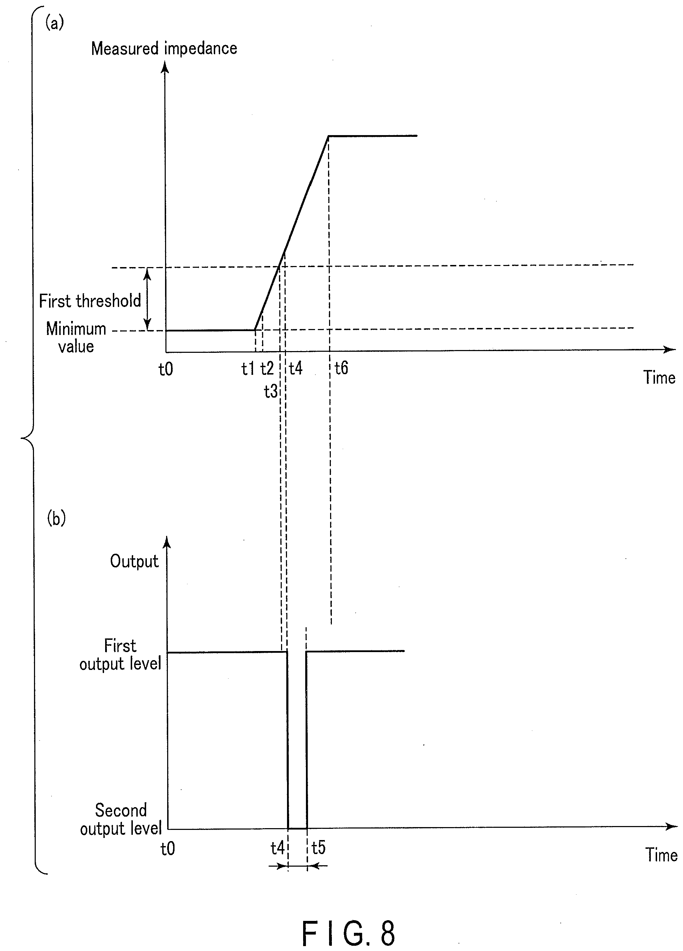

With reference to FIG. 8, the temporal change of measured impedance and that of output will be described. An upper figure (a) in FIG. 8 schematically shows values of measured impedance with respect to the passage of time, and a lower figure (b) in FIG. 8 schematically shows values of output of the output unit 140 with respect to the passage of time.

Let us assume that the output switch is on at time t0. At this point, the output level is set to the first output level as shown in the lower figure (b) in FIG. 8 by the processing of step S105 described above. It is assumed that the first electrode 212 and the living tissue 900 are in contact with each other at this time. Accordingly, the measured impedance Zmeas acquired by the above processing of step S106 is showing a low value. This value is stored as the minimum value Zmin of impedance.

After time t1, the first electrode 212 and the living tissue gradually move apart. At this time, an electric discharge occurs between the first electrode 212 and the living tissue 900. The measured impedance Zmeas gradually rises. For example, the measured impedance Zmeas at time t2 is higher than the impedance minimum value Zmin.

Suppose that the difference between the measured impedance Zmeas and the minimum value Zmin reaches the first threshold at time t3. At time t4 after this time t3, the output is changed to the second output level as shown in the lower figure (b) in FIG. 8 by the processing of step S110 described above. This instance shows the case where the second output level is zero. Note that the change to the second output level may be done at the time t3.

Here, the setting of the first threshold enables adjustment of sensitivity for the transition to the blanking period. That is, adopting a smaller first threshold increases the sensitivity and adopting a larger first threshold decreases the sensitivity. This first threshold may be set as appropriate.

After the time t4, the measured impedance Zmeas rises further since the first electrode 212 and the living tissue 900 move further away from each other. The blanking period for which the output is at the second output level is determined by the processing of step S111 and step S112 described above. The time at which the blanking period has passed is given as time t5. At the time t5, the output is changed to the first output level by the processing of step S105 described above. After time advances further, and from time t6 and onward, the first electrode 212 and the living tissue 900 are sufficiently distant from each other and the measured impedance Zmeas becomes a sufficiently large value.

As such, the time t0 to time t1 represents a period for performing treatment such as incision and hemostasis, and the time t6 and onward represent a period where the treatment is not performed, and the period from the time t1 to time t6 is a transition period where the first electrode 212 moves away from the living tissue 900. It has been known that at some point during this transition period, the output value could instantaneously deviate from a target value to a large extent due to the unintentional occurrence of a large electric discharge between the first electrode 212 and the living tissue 900, or the like. This embodiment, as described above, detects the first electrode 212 moving away from the living tissue 900 based on the impedance measurement and temporarily suppresses output for a certain time during the transition period. With this temporal output suppression, the output value is prevented from instantaneously deviating from a target value to a large extent.

Hereinafter, some modification examples of the embodiment will be provided.

### [Regarding the output level]

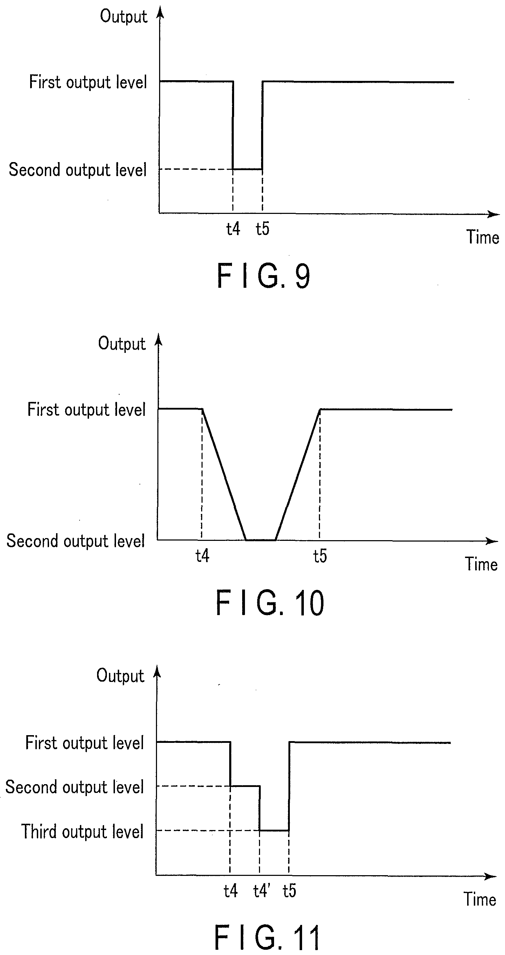

A modification example will be given in relation to the output for the above described blanking period from the time t4 to the time t5. The foregoing embodiment has assumed the case where the output value of the second output level is zero, that is, the output is halted, for the blanking period. However, the second output level for the blanking period is not limited to this, but may take any value as long as it is smaller than the first output level before and after the blanking period and it does not largely deviate from the target value. For example, as shown in FIG. 9, the second output level may be a value smaller than the first output level and larger than zero. As such, the output during the blanking period would suffice as long as it is at the second output level lower than the first output level, inclusive of zero.

Also, instead of suddenly changing the output from the first output level to the second output level for the blanking period as in the above embodiment, the power supply device 100 may gradually change the output from the first output level to the second output level as shown in FIG. 10. Also, the power supply device 100 may gradually change the output from the second output level to the first output level. Generally, such devices involve large output levels, so a rapid change of output levels often generates electric noise. Accordingly, a noise reduction effect can be expected by changing the output levels gradually.

Also, the above embodiment has given an example where the output level is changed between the first output level and the second output level for the blanking period, but this is not a limitation. For example, the blanking period may be divided into multiple portions as shown in FIG. 11. That is, the power supply device 100 changes the output level from a first output level to a second output level when a predetermined condition is satisfied. Furthermore, the power supply device 100 changes the output level from the second output level to a third output level when another predetermined condition is satisfied. Also, the power supply device 100 changes the output level from the third output level to the first output level when still another predetermined condition is satisfied. The power supply device 100 may also change the output level in a stepwise manner with three or more stages. The power supply device 100 may gradually lower the output level or change it in other patterns, too.

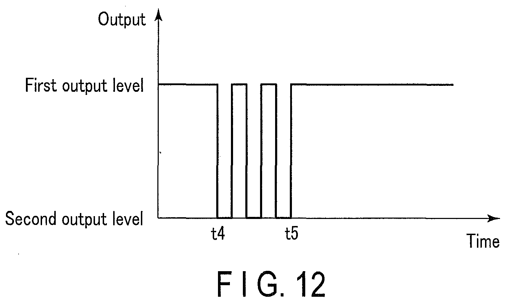

Also, as shown in FIG. 12, during the blanking period, the power supply device 100 may change the output level alternately at any number of times between a first output level and a second output level lower than the first output level. In this instance, instead of repeating the second output level and the first output level, the second output level and a third output level equal to or lower than the first output level may be repeated for the sake of reduction of noise generation as above. Frequent changes of the output level in this manner can suppress the output value instantaneously deviating significantly from a target value. Also, various patterns as can be obtained by combining the patterns of the output level changes described with reference to FIG. 9 to FIG. 12 may be adopted.

### [Regarding the setting of the blanking period]

The blanking period is not limited to the subject of determination at a predetermined time as in the embodiment described above. For example, it may be configured so that the output level is changed to the first output level when the measured impedance exceeds a predetermined value. With such a configuration, the output level can be lowered to the second output level when the living tissue 900 and the first electrode 212 are within a predetermined distance range, no matter how fast a user moves the first electrode 212.

### [Regarding the distance acquisition method]

The above embodiment has given an example where a distance between the living tissue 900 and the first electrode 212 is estimated based on the impedance of a circuit. The distance between the living tissue 900 and the first electrode 212 may be derived from information other than the impedance of a circuit. For example, the distance between the living tissue 900 and the first electrode 212 may be acquired based on current values or voltage values relating to the output. Also, the distance between the living tissue 900 and the first electrode 212 may be acquired based on images obtained by, for example, an imaging device provided for observing a treatment subject site. In this instance, the distance information acquisition unit 112 may include an image analyzing function. Also, distance measurement methods which utilize, for example, light or sound waves may be adopted. In this instance, the distance information acquisition unit 112 acquires the distance between the living tissue 900 and the first electrode 212 using light or sound waves.

### [Regarding the determination for the start of the blanking period]

According to the embodiment above, the blanking period is initiated when the difference Zmeas-Zmin obtained by subtracting the impedance minimum value Zmin from the measured impedance Zmeas is larger than the predetermined first threshold. However, the condition is not limited to this. Supposing that a maximum value of impedance measured when the first electrode 212 and the living tissue 900 are in contact with each other is given as Zmax, the blanking period may be configured so that it is initiated when the absolute value of the difference Zmeas-Zmax obtained by subtracting the impedance maximum value Zmax from the measured impedance Zmeas is larger than a predetermined first threshold. Also, supposing that an average value of impedance measured when the first electrode 212 and the living tissue 900 are in contact with each other is given as Zaverage, the blanking period may be configured so that it is initiated when the difference Zmeas-Zaverage obtained by subtracting the impedance average value Zaverage from the measured impedance Zmeas is larger than a predetermined first threshold. In this manner, the condition can be suitably changed as long as the blanking period is configured so that it is initiated upon measuring a value larger than the impedance measured when the first electrode 212 and the living tissue 900 are in contact with each other.

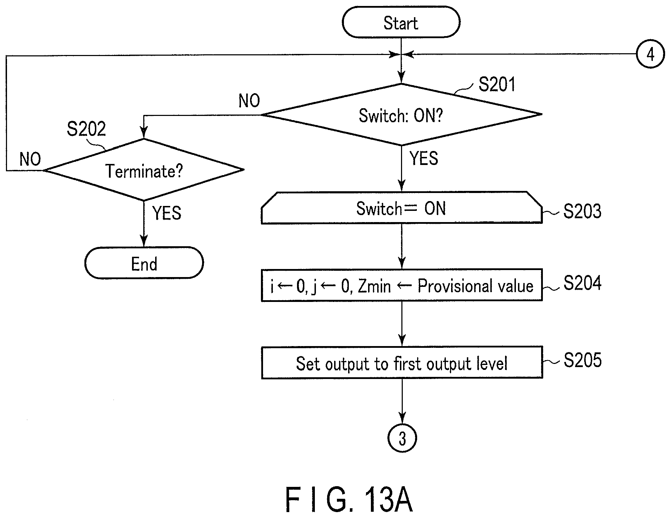

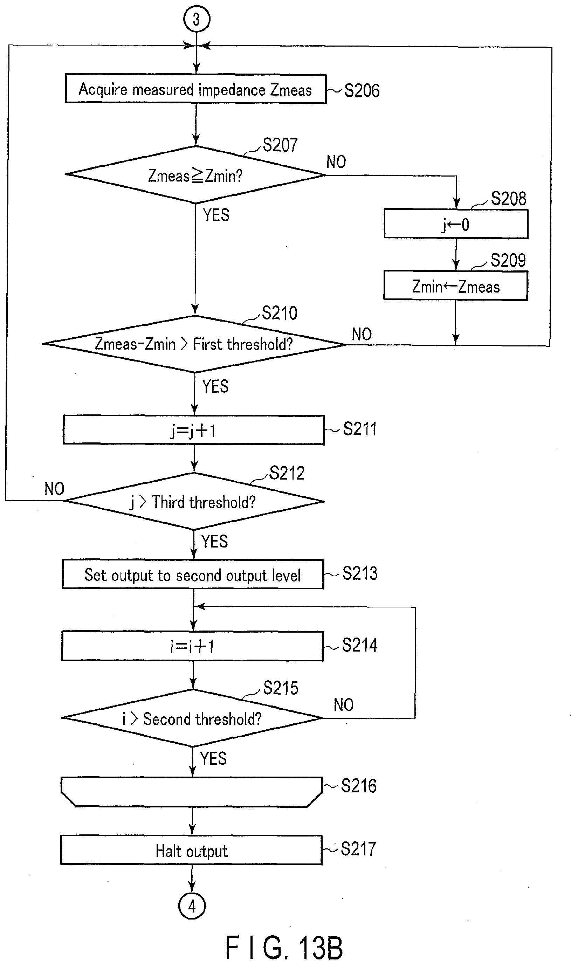

According to the above example, the blanking period is initiated when the absolute value of the difference Zmeas-Zmin is larger than the predetermined first threshold. However, if the blanking period is to be initiated upon satisfaction of the condition even once, a malfunction could occur due to the influence of noise, etc. Accordingly, the power supply device 100 may be configured so that the blanking period is initiated upon satisfaction of the condition a certain number of times. The processing in this instance will be described with reference to the flowcharts shown in FIG. 13A and FIG. 13B.

The operation of step S201 to step S203 is the same as the processing in step S101 to step S103 in the above embodiment. To briefly explain, the controller 110 determines at step S201 whether the output switch 250 is on or not. If it is not on, the processing proceeds to step S202. At step S202, it is determined whether or not to terminate the processing. If termination is determined, the processing ends. On the other hand, if termination is not determined, the processing returns to step S201. If it is determined at step S201 that the output switch 250 is on, the processing proceeds to step S203.

The processing from step S203 to step S216 is repetitive processing. The condition for repeating is that the output switch 250 is on. When the output switch is turned off, the processing comes out of this repetitive processing and proceeds to step S217.

At step S204, the controller 110 initializes variables stored in the storage 122. Here, it sets the first counter i for obtaining the blanking period to zero, and additionally sets a second counter j for avoiding false detection to zero. It also sets the impedance minimum value Zmin to a provisional value.

The operation of step S205 to step S207 is the same as the processing in step S105 to step S107 in the above embodiment. To briefly explain, the controller 110 at step S205 sets the level of output from the output unit 140 to the first output level. At step S206, the controller 110 acquires the measured impedance Zmeas.

At step S207, the controller 110 determines whether or not the measured impedance Zmeas is equal to or larger than the present minimum value Zmin. If the measured impedance Zmeas is smaller than the minimum value Zmin, the processing proceeds to step S208.

At step S208, the controller 110 resets the value of the second counter j to zero. Subsequently at step S209, the controller 110 sets the minimum value Zmin to the measured impedance Zmeas. The processing then returns to step S206.

At step S207, if the measured impedance Zmeas is determined to be equal to or larger than the minimum value Zmin, the processing proceeds to step S210. At step S210, the controller 110 determines whether or not the difference Zmeas-Zmin obtained by subtracting the impedance minimum value Zmin from the measured impedance Zmeas is larger than the predetermined first threshold. If the difference Zmeas-Zmin is not larger than the first threshold, the processing returns to step S206. If the difference Zmeas-Zmin is larger than the first threshold, the processing proceeds to step S211.

At step S211, the controller 110 increases the value of the second counter j stored in the storage 122.

At step S212, the controller 110 determines whether or not the second counter j is larger than a predetermined third threshold. If the second counter j is not larger than the third threshold, the processing returns to step S206. On the other hand, if the second counter j is larger than the third threshold, the processing proceeds to step S213.

According to this modification example as such, at step S210, when the number of times that the difference Zmeas-Zmin (which is obtained by subtracting the impedance minimum value Zmin from the measured impedance Zmeas) is determined to be larger than the first threshold exceeds the third threshold, the processing proceeds to step S213 for the first time. By proceeding to step S213 upon repeatedly determining that the difference Zmeas-Zmin is larger than the first threshold in this manner, unintended processing induced by noise, etc. that would change the output levels can be prevented.

The processing at step S213 to step S217 is the same as the processing in step S110 to step S114 in the above embodiment. To briefly explain, the controller 110 at step S213 sets the level of output from the output unit 140 to the second output level. At step S214, the controller 110 increases the first counter i. At step S215, the controller 110 determines whether or not the first counter i is larger than the predetermined second threshold. If the first counter i is not larger than the second threshold, the processing returns to step S214. That is, the processing of step S214 and step S215 is repeated until the first counter i exceeds the second threshold. At step S215, if the first counter i is determined to be larger than the second threshold, the processing proceeds to step S216. In other words, when the switch is on, the processing from step S203 is repeated.

According to this modification example, the sensitivity can be adjusted by providing the third threshold shown in FIG. 13B. While this example has adopted a determination criterion of whether or not the difference Zmeas-Zmin obtained by subtracting the impedance minimum value Zmin from the measured impedance Zmeas is larger than the predetermined first threshold, this is not a limitation. For example, whether an absolute value of the measured impedance Zmeas satisfies a predetermined condition or not may also be adopted as a determination criterion.

### [Regarding the high frequency treatment instrument]

The above embodiment has given an example where the high frequency treatment instrument 220 is a monopolar-type high frequency treatment instrument, but the high frequency treatment instrument 220 may also be a bipolar-type treatment instrument. In this case, two electrodes comprised by the treatment instrument would correspond to the first electrode 212 and the second electrode 214.

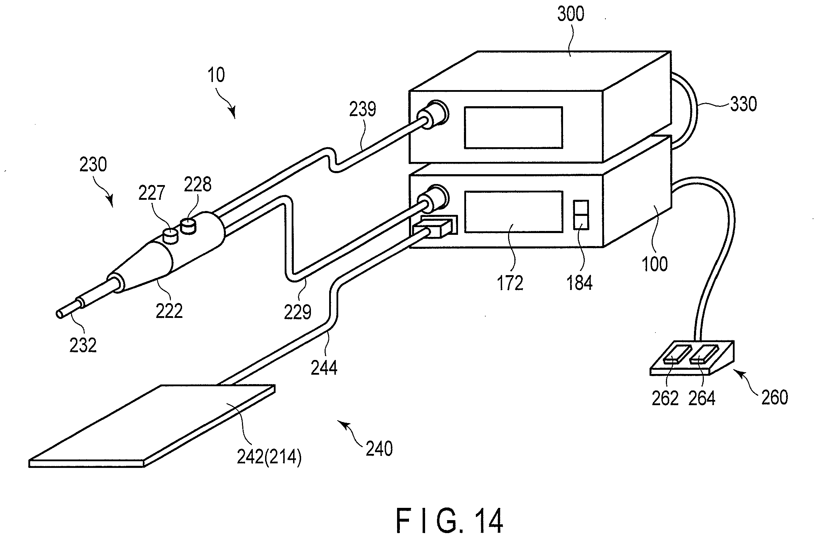

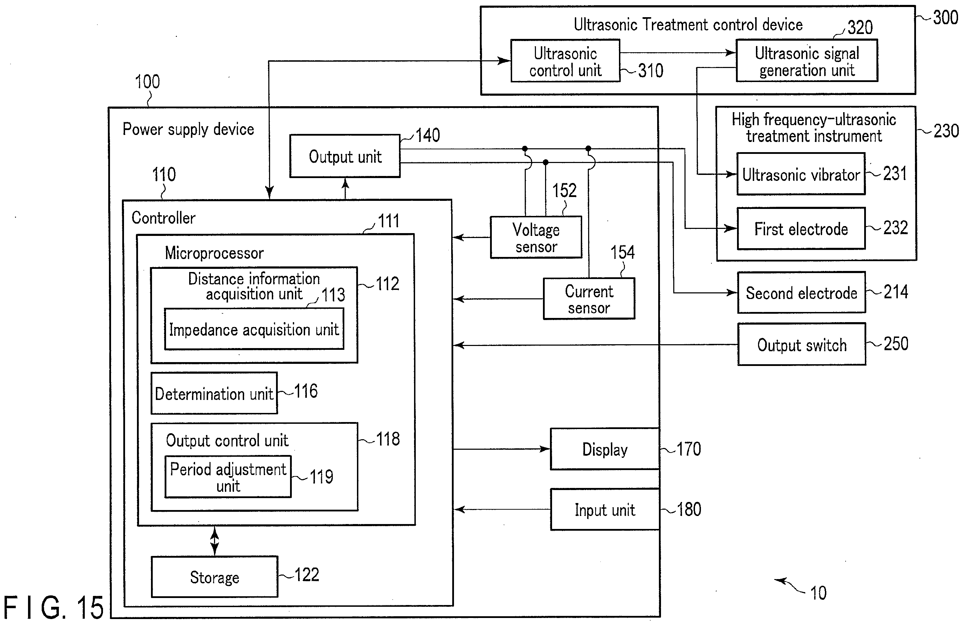

In the above embodiment, the high frequency treatment instrument 220 has been described as an instrument performing only the treatment with high frequency power, but is not limited to this. The treatment instrument may also be an instrument that comprises a probe capable of ultrasonic vibration and utilizes both high frequency energy and ultrasonic energy to treat a treatment subject. A modification example relating to such a high frequency-ultrasonic treatment system 10 that utilizes both high frequency energy and ultrasonic energy will be described with reference to FIG. 14 and FIG. 15. Here, descriptions will be made of the differences from the above embodiment, and overlapping descriptions for the same portions will be omitted by using the same reference signs.

FIG. 14 schematically shows an external view of the high frequency-ultrasonic treatment system 10 according to this modification example. Also, FIG. 15 schematically shows a configuration example of the high frequency-ultrasonic treatment system 10 according to this modification example. The high frequency-ultrasonic treatment system 10 according to this modification example comprises a high frequency-ultrasonic treatment instrument 230 in place of the high frequency treatment instrument 220 of the above embodiment. The high frequency-ultrasonic treatment instrument 230 comprises a first electrode 232 corresponding to the first electrode 212 according to the above embodiment. The high frequency-ultrasonic treatment instrument 230 further comprises an ultrasonic vibrator 231. The ultrasonic vibrator 231 is a source of vibration and causes the first electrode 232 to ultrasonically vibrate. That is, the first electrode 232 functions as both the electrode of a high frequency treatment instrument and the probe of an ultrasonic treatment instrument.

A power supply device 100, a second electrode 214 that functions as a return electrode 242, and an output switch 250 according to this modification example have the same configuration as the power supply device 100, the second electrode 214, and the output switch 250 of the above embodiment, respectively. In this modification example, the high frequency-ultrasonic treatment system 10 comprises an ultrasonic treatment control device 300 for controlling operations of the ultrasonic vibrator 231, in addition to the power supply device 100. The ultrasonic treatment control device 300 may be provided in the power supply device 100.

The ultrasonic treatment control device 300 is connected to the power supply device 100 via a cable 330. Also, the ultrasonic treatment control device 300 is connected to the high frequency-ultrasonic treatment instrument 230 via a cable 239. The ultrasonic treatment control device 300 comprises an ultrasonic control unit 310 and an ultrasonic signal generation unit 320. The ultrasonic control unit 310 controls operations of each component of the ultrasonic treatment control device 300 including the ultrasonic signal generation unit 320. Also, the ultrasonic control unit 310 is connected to the controller 110 of the power supply device 100 and exchanges necessary information with the controller 110. The ultrasonic signal generation unit 320 generates signals to drive the ultrasonic vibrator 231 under the control of the ultrasonic control unit 310.

In the treatment with the high frequency-ultrasonic treatment instrument 230, a user brings the first electrode 232 into contact with living tissue 900 as a treatment subject and turns the output switch 250 on. At this time, the high frequency-ultrasonic treatment instrument 230 outputs energy. For example, when a first switch 227 of the output switch 250 is turned on, the ultrasonic control unit 310, having obtained the information about the turning on of the first switch 227 through the controller 110, causes the ultrasonic signal generation unit 320 to output a signal for ultrasonic generation. With this output signal, the ultrasonic vibrator 231 ultrasonically vibrates, and this vibration is transmitted for the first electrode 232 to ultrasonically vibrate. Concurrently, the controller 110 causes an output unit 140 to output high frequency power. As a result, a high frequency current flows through the living tissue 900 between the first electrode 232 and the second electrode 214. Friction between the living tissue 900 and the ultrasonically vibrating first electrode 232 generates heat. Also, the high frequency current flowing through the living tissue 900 generates heat in the living tissue 900. With these types of heat, incision or hemostasis of the living tissue 900 is performed.

On the other hand, for example, when a second switch 228 of the output switch 250 is turned on, only the output of high frequency power by the output unit 140 is performed and the ultrasonic signal generation unit 320 does not output a signal for ultrasonic generation. As a result, a high frequency current flows through the living tissue 900 between the first electrode 232 and the second electrode 214 to generate heat. With this heat, the living tissue 900 undergoes, for example, hemostatic treatment.

The concurrent application of the ultrasonic vibration energy and the high frequency energy via the first electrode 232 to the living tissue 900 as a treatment subject can suppress the living tissue sticking to the first electrode 232. As a result, smooth incision or hemostasis of the living tissue 900 can be achieved.

It is generally known that the application of ultrasonic vibration to living tissue 900 would cause a small portion of the living tissue 900 to scatter in the form of a mist. In particular, if a treatment subject, i.e. the living tissue 900, has a large content of fat, the fat scatters in the form of a mist during treatment. If the distance between the first electrode 232 and the living tissue 900 reaches a certain distance and the output level of the high frequency power becomes high while the scattered mist of fat is present around the treatment site, an unintentionally large electric discharge can easily occur. As in the above embodiment, the high frequency-ultrasonic treatment system 10 according to this modification example also detects the first electrode 232 moving away from the living tissue 900 and temporarily suppresses the high frequency power output for a certain time during the transition period. With this temporal output suppression, the output value is prevented from instantaneously deviating from a target value to a large extent due to the occurrence of an unintentionally large electric discharge, even if there is floating mist of fat. As such, the function of temporarily suppressing the high frequency power output is particularly effective when the treatment with ultrasonic vibration is performed together with the treatment with high frequency power.

## Claims

1. A power supply device for a high frequency treatment instrument which treats living tissue by supplying high frequency power to the living tissue using an electrode, the power supply device comprising:
an output unit which supplies the high frequency power to the electrode;
a distance information acquisition unit which acquires distance information for a distance between the living tissue and the electrode;
a determination unit which determines whether or not the distance information satisfies a first condition when the distance between the living tissue and the electrode is increasing; and
an output control unit which controls the output unit so that output by the output unit is placed in a controlled state if the distance information satisfies the first condition and so that the output is set to a first output level higher than an output level in the controlled state if a second condition is satisfied after the output is placed in the controlled state.

2. The power supply device according to claim 1, wherein the distance information acquisition unit acquires, as the distance information, an impedance measured based on the output by the output unit.

3. The power supply device according to claim 2, wherein the determination unit determines that the first condition is satisfied if the impedance exceeds a value measured when the living tissue and the electrode are in contact with each other by a predetermined value.

4. The power supply device according to claim 2, wherein the determination unit obtains the impedance, holds a minimum value of the impedance, and determines that the first condition is satisfied if a difference between a present value of the impedance and the minimum value of the impedance exceeds a first threshold.

5. The power supply device according to claim 2, wherein the determination unit obtains the impedance, holds a minimum value of the impedance, and determines that the first condition is satisfied if a difference between a present value of the impedance and the minimum value of the impedance exceeds a first threshold a predetermined number of times.

6. The power supply device according to claim 2, wherein the output control unit determines that the first condition is satisfied if the impedance exceeds a predetermined threshold.

7. The power supply device according to claim 1, wherein the output control unit determines that the second condition is satisfied if a predetermined period has passed after the output is placed in the controlled state.

8. The power supply device according to claim 1, wherein the controlled state is a state in which the output is set to a second output level lower than the first output level.

9. The power supply device according to claim 8, wherein the output control unit gradually changes the output.

10. The power supply device according to claim 1, wherein the controlled state comprises a state in which the output is set to a second output level lower than the first output level, and a state in which the output is set to a third output level equal to or lower than the first output level, the states being repetitive.

11. The power supply device according to claim 1, wherein the output control unit controls the output using a power value, a voltage value, or a current value.

12. The power supply device according to claim 1, further comprising an ultrasonic treatment control device for ultrasonically vibrating the high frequency treatment instrument.

13. A high frequency treatment system comprising:
the power supply device according to claim 1; and
the high frequency treatment instrument.

14. The high frequency treatment system according to claim 13, wherein the high frequency treatment instrument is a high frequency-ultrasonic treatment instrument and is further configured to treat the living tissue by ultrasonic vibration.

15. A method for operating a high frequency treatment system which treats living tissue by supplying high frequency power to the living tissue using an electrode, the method comprising:
supplying, by an output unit, the high frequency power to the electrode;
acquiring, by a distance information acquisition unit, distance information for a distance between the living tissue and the electrode;
determining, by a determination unit, whether or not the distance information satisfies a first condition when the distance between the living tissue and the electrode is increasing; and
controlling, by an output control unit, output of the high frequency power so that the output is placed in a controlled state if the distance information satisfies the first condition and so that the output is set to a first output level higher than an output level in the controlled state if a second condition is satisfied after the output is placed in the controlled state.
